(19) Europäisches Patentamt
European Patent Office
Office européen des brevets

(11) Publication number: **0 204 009 B1**

# (12) EUROPEAN PATENT SPECIFICATION

(45) Date of publication of patent specification: **05.02.92**

(51) Int. Cl.⁵: **C12N 1/14**, C12P 7/02, C07D 307/92, //(C12N1/14, C12R1:645)

(21) Application number: **85106304.0**

(22) Date of filing: **22.05.85**

## (54) Process for producing diol and furan and microorganism capable of same.

(43) Date of publication of application:
**10.12.86 Bulletin 86/50**

(45) Publication of the grant of the patent:
**05.02.92 Bulletin 92/06**

(84) Designated Contracting States:
**AT BE CH DE FR GB IT LI NL SE**

(56) References cited:

**CHEMICAL ABSTRACTS, vol. 95, 1981, page 341, no. 200224z, Columbus, Ohio, US; G.S. DE HOOG et al.: "Hyphozyma, a new genus of yeast-like hyphomycetes", & ANTONIE VAN LEEUWENHOEK 1981, 47(4), 339-52**

**BIOLOGICAL ABSTRACTS, vol. 78, no. 7, 1984, page 5518, no. 49040, Biosciences Information Service, Philadelphia, PA, US; A. KOCKOVA-KRATOCHVILOVA et al.: "Taxonomic characteristics of yeasts isolated from bath facilities", & CESKA MYKOL, 38(1), 11-20, 1984**

(73) Proprietor: **BASF K & F Corporation**
**100 Cherry Hill Road**
**Parsippany, NJ 07054(US)**

(72) Inventor: **Farbood, Mohamad I.**
**111 Washington Avenue**
**West Windsor New Jersey 08561(US)**
Inventor: **Willis, Brian J.**
**Bergenfield Mill Lane**
**Nonington, Near Dover Kent(GB)**
Inventor: **Christenson, Philip A.**
**118 Busteed Drive**
**Midland Park New Jersey 07432(US)**

(74) Representative: **Vossius & Partner**
**Siebertstrasse 4 P.O. Box 86 07 67**
**W-8000 München 86(DE)**

EP 0 204 009 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 99, 1983, page 642, no. 22720f, Columbus, Ohio, US; P.F. VLAD et al.: "New convenient methods for the preparation of tetrahydrofurans from 1,4-diols", & SYNTHESIS 1983, (3), 216-19

CHEMICAL ABSTRACTS, vol. 93, 1980, page 756, no. 114751w, Columbus, Ohio, US; V.E. SIBIERTSEVA et al.: "Industrial method for producing ambroxide", & MASLO-ZHIR. PROM-ST. 1979, (12),25-6

CHEMICAL ABSTRACTS, vol. 102, 1985, page 347, n. 12195e, Columbus, Ohio, US; V.E. SIBIRTSEVA et al.: "By-products in the synthesis of ambroxide", & MASLO-ZHIR. PROM-ST. 1984, (5), 21-2

Antonie van Leeurvenhoek, Vol. 47, 1981, pp. 339-352

Ceska Mykol, 38(1), 1984, pp. 11-20

## Description

Dodecahydro-3a,6,6,9a-tetramethylnaphtho[2,1-b]furan (1) is an important fragrance chemical (see U.S. Patent 3,029,255). It has been used in high quality perfume compositions and in functional products, such as fragrances for toiletries and household products, where a persistent amber effect is desired. Compound 1 is also a component of tincture of ambergris [see B.D. Mookherjee and R.R. Patel, Proceedings of the 7th International Congress of Essential Oils, Kyoto, Japan, (1977), paper number 136], and synthetic 1 has been used in artificial ambergris formulations. Compound 1 may be manufactured from 2-ethenyldecahydro-2-hydroxy-α-2,5,5,8a-pentamethyl-1-naphthalenepropanol (4), commonly referred to as Sclareol, obtained from Clary Sage (Salvia Sclarea).

U.S. Patent 3,050,532 discloses a method of converting Compound 4 into dodecahydro-3a,6,6,9a-tetramethylnaphth[2,1-b]furan-2(1H)-one (2) using a two-stage oxidation sequence. In the first step an aqueous dispersion of Sclareol is intimately contacted with an alkali metal permanganate oxidizing agent, under alkaline conditions, to partially oxidize the Sclareol. During the second step the resulting aqueous reaction mixture from the first step is acidified and intimately contacted with a permanaganate or chromic acid oxidizing agent under acid conditions, thereby completing the oxidation.

Compound 2 may be readily converted to Compound 1 by known methods. For example, reducing Compound 2 with hydride reagents (see for example, Helv. Chim. Acta 1950, 33, 1308) provides decahydro-2-hydroxy-2,5,5,8a-tetramethylnaphthaleneethanol (3) which is readily converted by cyclization to Compound 1.

U.S. Patent 3,029,255 discloses a method for making Compound 1 by dehydrating Compound 3 with $Al_2O_3$ at 200-225 °C, followed by heating in vacuo in the presence of β-naphthalene sulfonic acid (130 °C up to 160 °C) to effect cyclization to Compound 1.

Alternatively, Compound 1 may be obtained by cyclization of Compound 3 using toluene-p-sulfonyl chloride in pyridine, as disclosed by Cambie et al. (see Aust. J. Chem., 1971, 24, 591).

There is no teaching or suggestion in the prior art of converting labdane compounds via microbiological methods into decahydro-2-hydroxy-α,2,5,5,8a-tetramethylnaphthaleneethanol (3) or dodecahydro-3a,6,6,9a-tetramethylnaphtho[2,1-b]furan (1), according to the novel and commercially efficient process provided by this invention.

The present invention concerns a biologically pure culture of the microorganism Hyphozyma roseoniger ATCC 20624.

The present invention further concerns a biologically pure culture of a Hyphozyma roseoniger microorganism or of a mutant thereof, which has the identifying characteristic of Hyphozyma roseoniger ATCC 20624 of being capable of converting labdane compounds into recoverable quantities of a diol of the formula (3)

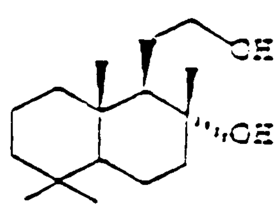

(3)

or into recoverable quantities of a furan of the formula (1)

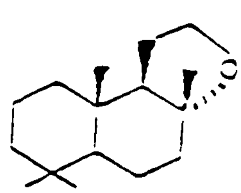

(1)

wherein the labdane compound is

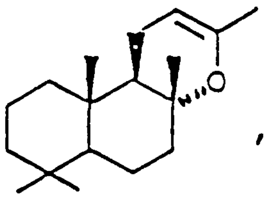,

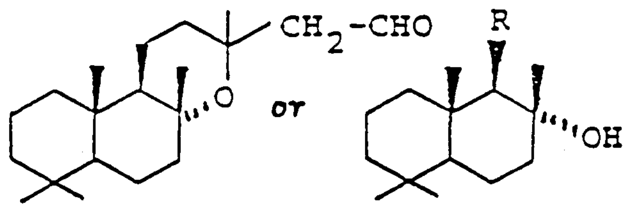

or

wherein R is

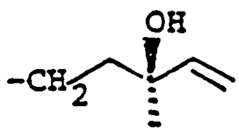

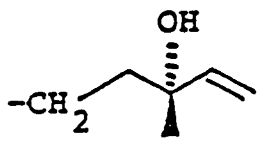

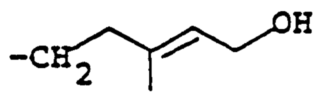

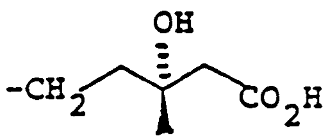

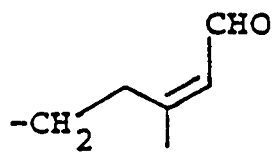

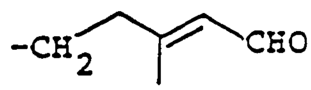

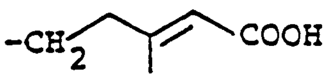

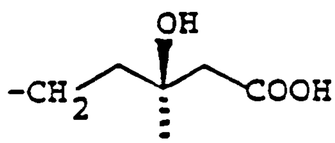

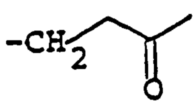

or

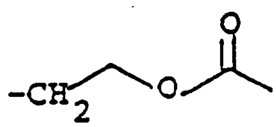

.

The culture may be lyophilized.

In another embodiment, the present invention concerns a mixture prepared by cultivating one of the above characterized biologically pure cultures under aerobic conditions in an aqueous nutrient medium.

The cultivation of the microorganism may be performed at a pH of between about 2.5 and about 9.0 and at a temperature of about 12° C and about 30° C.

In a further embodiment, the present invention concerns a process for preparing a diol having the structure

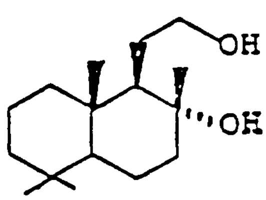

(3)

which comprises cultivating one of the above characterized biologically pure cultures under aerobic conditions in an aqueous nutrient medium containing one or more of the compounds

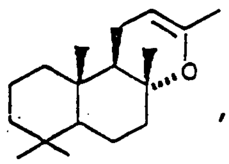 ,

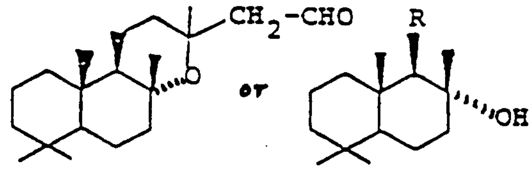

or

wherein R is

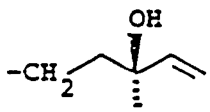

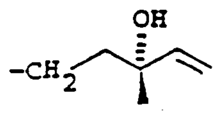

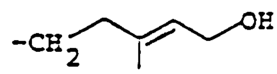

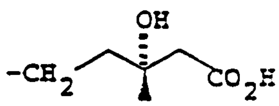

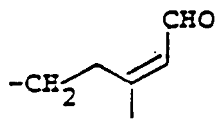

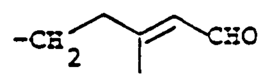

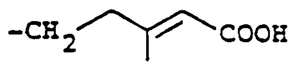

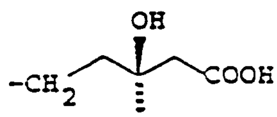

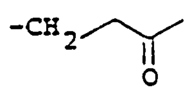

or

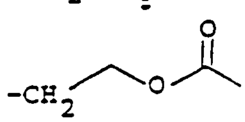

The cultivation of the microorganism may be performed at a pH of between about 2.5 and about 9.0 and at a temperature of about 12 °C and about 30 °C.

In a still further embodiment, the present invention concerns a process for preparing a furan compound having the structure

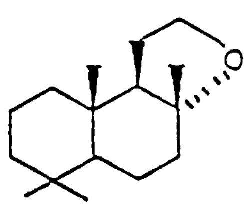

(1)

which comprises cultivating one of the above characterized biologically pure cultures under aerobic conditions in an aqueous nutrient medium containing one or more compounds from the group consisting of

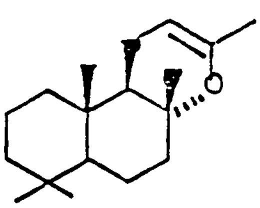 ,

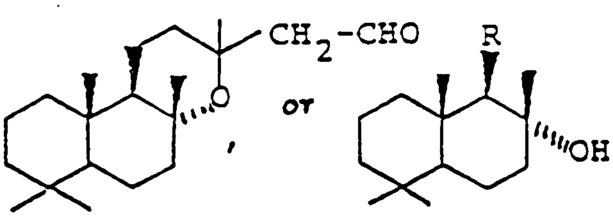

, or

wherein R is

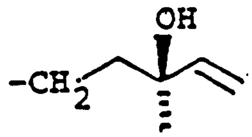
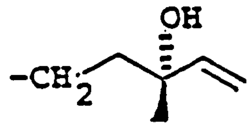
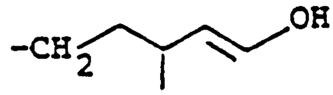
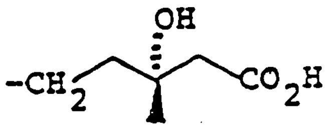
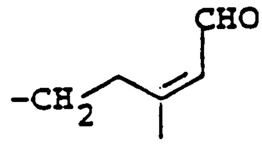
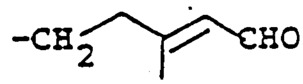
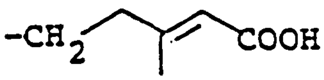
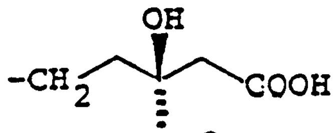
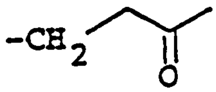
or
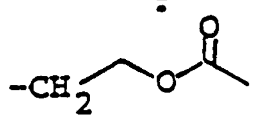

to form a diol having the structure

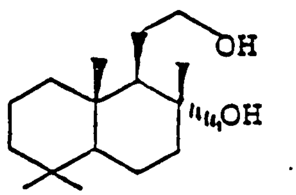

The diol obtained may be cyclized in the aqueous nutrient medium to form said furan compound which is then recovered. In another embodiment the diol may be separated from the aqueous nutrient medium and then cyclized to form said furan compound which is then recovered.

The structures of some of the compounds of interest in the present invention are given below.

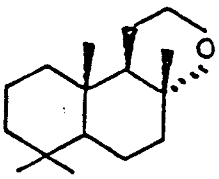

1

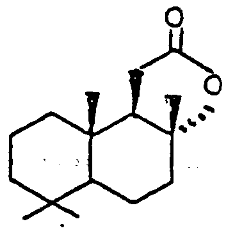

2

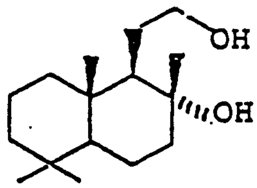

3

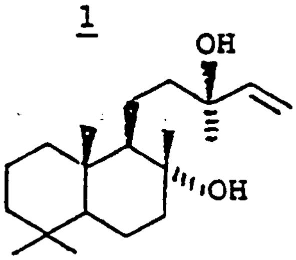

4

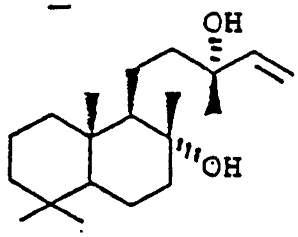

4

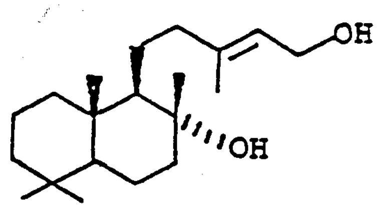

6

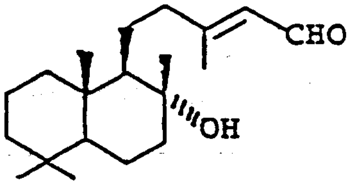

7

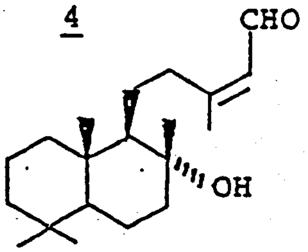

8

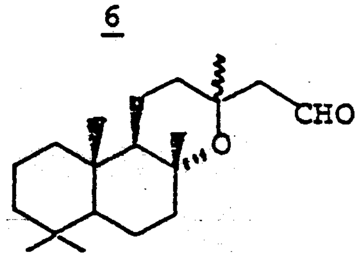

9

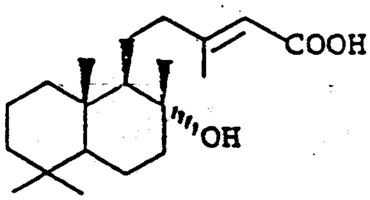

10

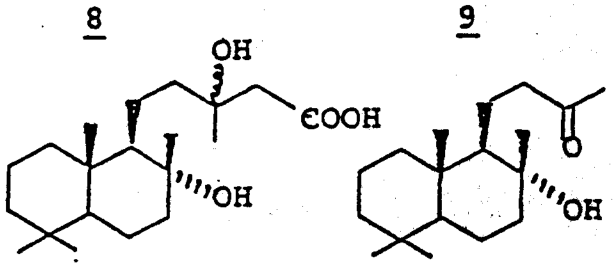

11

12

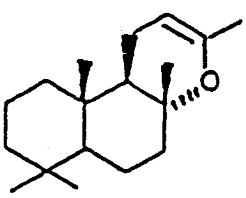

13

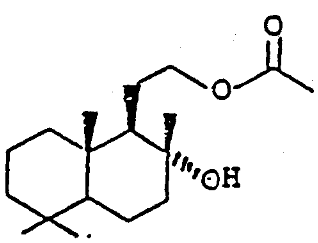

14

Compounds 4 through 14 may be used as substrates in the transformation process for the present invention to produce the desired products. Compounds 6, 11, and 13 have been observed as intermediates during the transformation of Sclareol according to the process of this invention. The transformation process involves cultivation of the microorganism Hyphozyma roseoniger, CBS 214.83 and ATCC 20624 in an aqueous nutrient medium in the presence of the compounds 4 through 14. These compounds may be used singularly or as a mixture containing any number of said compounds.

The form in which the microorganisms are used is not critical. They can be used as the culture (suspension), i.e. including the cells and the corresponding nutrient solution, or in the form of cells suspended in a buffer solution. The cells, or an enzyme extract thereof, may be immobilized on a suitable solid support, which may then be used to effect transformations.

The suspended culture mixture is prepared by inoculation of a suitable aqueous nutrient medium with the microorganism. A suitable nutrient medium is one which contains nitrogen sources, inorganic salts, growth factors, the desired substrate(s), and optionally other carbon sources. Some carbon sources suitable

for use in the inventive process include, for example, glucose, galactose, L-sorbose, maltose, sucrose, cellobiose, trehalose, L-arabinose, L-rhamnose, ethanol, glycerol, L-erythrithol, D-mannitol, lactose, melibiose, raffinose, melezitose, starch, D-xylose, D-sorbitol, α-methyl-D-glucoside, lactic acid, citric acid and succinic acid. Suitable nitrogen sources include, for example, nitrogen-containing organic substances such as peptone, meat extract, yeast extract, corn steep liquor, casein, urea, amino acids, or nitrogen-containing inorganic compounds such as nitrates, nitrites and inorganic ammonium salts Suitable inorganic salts include, for example, phosphates of magnesium, potassium, calcium, or sodium. The above mentioned culture medium nutrients may be supplemented with, for example, one or more vitamins of the B group and/or one or more trace minerals such as Fe, Mo, Cu, Mn, and B, as desired. The Vitamins or trace minerals are not necessary when a small amount of yeast extract is added to the medium. Addition of an antibiotic, such as chloroamphinical or chlorotetracycline, may be desirable when bacterial contamination is a problem.

The cultivation of the microorganism may be carried out as a stationary culture or as a submersed (e.g., shaking culture, fermentor culture) under aerobic conditions. One may suitably work in the pH range of from between about 2.5 and about 9.0, and preferably in the range of from between about 3.0 and about 7.5 and most preferably between about 3.0 and about 6.5 The pH may be regulated by the addition of inorganic or organic acids, such as hydrochloric acid, acetic acid, and oxalic acid, or by the addition of bases, such as sodium hydroxide, and ammonium hydroxide, or by the addition of a buffer, such as phosphate or phthalate. The incubation temperature should suitably be maintained between about 12° C and about 33° C, with a range between about 15° C and about 30° C being more preferred, and a range between about 18° C and about 28° C being most preferred.

The process in accordance with this invention may be conveniently carried out by adding one or more of the compounds 4 through 14 to the nutrient medium at the onset of cultivation, as the sole carbon source. Alternatively, the substrate may be added in combination with another carbon source, such as dextrose, either during cultivation, or when the carbon source is depleted. The only restriction on the concentration of substrate in the culture medium is that of being able to effectively aerate the culture. However, the substrate concentration is preferably in the range of between about 0.1 g/L and about 100g/L, more preferably in the range of between about 0.5 g/L and about 50 g/L, and most preferably in the range between about 1.5 g/L about 30 g/L. The transformation can be suitably carried under any of the above mentioned conditions.

The total transformation time (after initial cultivation period) may vary depending on the composition of the nutrient medium and the substrate concentration. In general, shaking flask cultures require from between about 12 hours and about 264 hours. However, when a fermentor is used the cultivation time may be reduced to about 48 hours or less.

The transformation may be carried out using the cells of the microorganism isolated from the culture solution, or with an enzyme extract isolated from the cells in a manner well known to the art. In this case, the transformation can be conveniently carried out in a variety of aqueous nutrient mediums including, for example, in a buffer solution, in a physiological salt solution, in a fresh nutrient solution, or in water. The isolated cells or enzyme extract may be immobilized on a solid support and the desired transformation effected. Also, transformation of the substrate may be effected by mutants of this organism. Such mutants can be readily obtained by methods well known in the art, for example, by exposing the cells to UV or X-rays, or known mutagenic substances, such as for example, acridine orange.

The substrate can be added to the medium as a powder, or a slurry in an emulsifier such as Tween-80 (polyoxyethylenesorbitan monostearate), or as a solution in an emulsifier, or as a solution in a hydrophilic solvent such as acetone, methanol, ethanol, ethylene glycol, or dioxan. A surface-active agent, or a dispersion agent can also be added to an aqueous suspension of the substrate, or the substrate can be emulsified using ultrasound.

Conventional antifoam agents, such as silicone oils (e.g., UCON), polyalkyleneglycol derivatives, maize oil, or soya oil, can be used to control foaming.

The transformation of the substrate can be monitored using standard analytical techniques such as GLC, TLC, HPLC, IR, and NMR. If a rapid disappearance of the substrate is observed more substrate can then be added, in order to maximize the transformation capacity of the microorganism. The process is generally terminated when most of the substrate has disappeared from the culture medium. Compound 3 may be recovered from the aqueous nutrient medium, or may be cyclized to the furan Compound 2,either in the aqueous nutrient medium,or after recovery. Isolation and purification of Compounds 1 or 3 from the fermentation broth may be achieved by conventional techniques including, filtration or centrifugation, solvent extraction, distillation, crystallization, and the like. Compound 3 may be converted to the furan Compound 1 by conventional cyclization methods well known in the art. For example, reaction of diol 3 with toluene -p-sulfonyl chloride in pyridine at 0° C according to the procedure described by Cambie et al. (see Aust. J. Chem., 1971, 24, 591), which is incorporated herein by reference. This procedure may optionally be employed either on the transformation mixture or on the recovered diol Compound 3. Examples of other cyclization methods are described by R.B. Wagner and H.D. Zook in "Synthetic Organic Chemistry", John Wiley, 1965, pp 838-839, which is incorporated herein by reference.

The microorganism employed in this invention was isolated from a soil sample obtained from central New Jersey, United States of America. This strain has been deposited with the Centraalbureau voor Schimmelculture and the American Type Culture Collection with the accession number CBS 214.83 and ATCC 20624, respectively.

The organism was studied and characterized by Centralbureau voor Schimmel Cultures (CBS). Due to the pink coloring of its colonies and its unique morphological and physiochemical properties, CBS has assigned the inventive microorganism the name Hyphozyma roseoniger.

This organism has distinct yeast and filamentous forms. Both forms exhibit similar biological properties and perform the transformations described herein.

The properties of the yeast phase of the said microorganism are described below:

1. Shape and Size

Growth on YM Agar: Pink, glistening, smooth colonies present filamentous growth cells are budding, round or cylindrical ca. 2 x 7 µm or sometimes larger.

Growth on Malt Extract Agar: Pink, occasionally brownish with formation of true hyphae after 2 to 3 weeks, with no clump connections, shiny.

Growth on Corn Meal Agar: Light orange-pink glistening. Smooth colonies fringed with mycelia.

Growth on Potato Dextrose Agar and Difco Malt Agar: Pink, smooth, glistening and occasionally turns black at room temperature after 2 to 3 weeks.

Growth on YPCA Agar: Attaining 8 mm diameter in 10 days, flat, slimy, pale orange (6A3; Kornerups Wanscher, 1978), with sharp, somewhat lobed margins.

Growth on ChA Agar: 4 mm diameter in 10 days. After 3 weeks centrally becoming olivaceous, with light brown (6D6) to dark brown (5F7), finally a submerged mycelium extending from the mucuous colony, leading to a dense, olive brown (4F4) colony locally with thin dirty,white central patches, later dense fascicles of aerial mycelium. The organism produces true hyphae with anastomoses observed on potato and rice slides. It seems to be a hyphomycets fungus with a distinct yeast phase in its life cycle.

2. Fermentation on Sugars (see Table 1)

TABLE 1

| Fermentation | | |
|---|---|---|
| Compound | Gas | Acid |
| Glucose | - | - |
| Galactose | - | - |
| Maltose | - | - |
| Sucrose | - | - |
| Lactose | - | - |
| Raffinose | - | - |
| Melibiose | - | - |
| Inulin | - | - |

3. Assimilation of Carbon Compounds (see Table 2)

TABLE 2

| Assimilation of Carbon Compounds | | | |
|---|---|---|---|
| Glucose | + | Lactose | v |
| Galactose | + | Melibiose | + |
| L-sorbose | + | Raffinose | + |
| Maltose | + | Melezitose | + |
| Sucrose | + | Inulin | - |
| Cellobiose | + | Soluble starch | + |
| Trehalose | + | D-xylose | + |
| L-arabinose | + | D-sorbitol | + |
| D-arabinose | - | α-Methyl-D-glucoside | + |
| D-ribose | + | Salicin | - |
| L-rhamnose | + | Inositol | - |
| D-glucosamine | - | Lactic acid | + |
| Ethanol | + | Citric acid | + |
| Glycerol | + | Succinic acid | + |
| L-erythrithol | + | Glucono-γ-lactone | + |
| Adonitol | - | | |
| Ducitol | - | | |
| D-mannitol | + | | |

4. Splitting Arbutin: positive
5. Assimilaton of $NH_4NO_3$: positive
6. Assimilation of $KNO_3$: positive
7. Assimilation of $KNO_2$: positive
8. Growth on Ethylamine: positive
9. Growth on Vitamin- free Medium: positive
10. Growth at 12°C: positive
11. Growth at 26°C: positive
12. Growth at 30°C: positive
13. Growth at 37°C: negative
14. Growth at 45°C: negative

The properties of the filamentous form of the said organism are similar to the yeast phase with the following exceptions:

Growth on YM Agar: Pink, rough colonies, presents filamentous growth with true hyphae.

Growth on Malt Agar or Potato Dextrose Agar: Pink, rough colonies, presents filamentous growth after 2 weeks at room temperature,colonies turn black.

Growth on Potato and Rice Slides: Produces true hyphae with anastomoses.

Growth in Liquid Medium such as YM Broth: Pink, presents yeast-like growth and occasionally some mycellium.

Growth on Potato Sucrose Agar: Filamentous with evidence of a yeast phase.

The following examples serve to illustrate embodiments of the invention as it is now preferred to practice it but in no way are meant to limit the scope thereof. Unless otherwise stated, weights are in grams, temperatures are in degrees centrigrade and pressure in mm Hg.

EXAMPLE 1

This example demonstrates the fermentation process using 2-ethenyldecahydro-2-hydroxy-α;2,5,5,8a-pentamethyl-1-naphthalene propanol (4) as substrate.

Four flasks, each containing an aqueous solution (100 mL) of 0.1% $NH_4NO_3$, 0.1% $H_2KPO_4$, 0.05% $MgSO_4.7H_2O$, trace minerals,and Vitamin B complex were sterilized at 120°C for 20 minutes. A 50% aqueous solution of dextrose (5 mL) and Tween-80 (0.1 mL), containing Sclareol (4) (10 mg) was added to each flask. Each flask was inoculated with 5% by volume of three days grown cells CBS 214.83 (ATCC 20624).The cultures were then incubated at 25 ± 1°C on a rotary shaker (200 rpm) for 3 to 4 days. After the initial incubation period a mixture of Sclareol dissolved in Tween-80 (8.0 g) was added in portions during the next 5 days, afterwhich the incubation was continued for a further 4 days. At the end of the incubation

period the contents of the four flasks were combined, extracted with ethyl acetate (3 x 200 mL) and dried ($Na_2SO_4$). Evaporation of the solvent provided a crude extract (4.0 g) , which was crystallized from hexane/chloroform to provide decahydro-2-hydroxy-2,5,5, 8a-tetramethylnaphthaleneethanol (3) (2.4 g), mp 130.5-131.5° C, (lit. 132-133° C) GLC purity 100%, H-NMR ($CDCl_3$) $\delta$ 0.79 (6H, 2s), 0.87 (3H, 2), (3H, 2), 0.9-20 (16H, m), 3.41-3.49 (1H, m), 3.72-3.79 (1H, m). IR ($CHCl_3$) $\nu_{max}$, 3580, 3360, 2950, 1460, 1380 cm. MS m/e 236, 221, 117, 137, 109. $[\alpha]_{22}^{D}$ = -16.8° ($CHCl_3$).

[Literature 132-133°C,

$$[\alpha]_D^{22.5} = -17.3° \ (CHCl_3).]$$

[see M. Stoll and M. Hinder Helv.Chim. Acta (1953) 36 1955-2008.]

EXAMPLE 2

This example demonstrates the efficacy of the fermentation process using different levels of Sclareol (4) as substrate.

A procedure similar to that described in Example 1 was used except that yeast extract (0.1 g) was substituted for the trace minerals and vitamins, and that the Sclareol (4) was recrystallized from hexanes, pulvarized, passed through a 50-mesh seive, and then mixed with an equal weight of Tween-80. After an initial incubation period of 4 days, the mixture of Sclareol (4) and Tween-80 was added to each flask incrementally over a period of 5 days and then incubated for an additional 4 days. Table 3 below shows the amount of Sclareol (4) added to each flask and the yield of isolated diol 3. Each product exhibited spectral data identical with that reported in Example 1.

Table 3

| Total Weight of Sclareol (4) Flask | g | Isolated Yield of Diol 3 % |
|---|---|---|
| 1 | 2 | 81 |
| 2 | 3 | 74 |
| 3 | 5 | 71 |

EXAMPLE 3

This example demonstrates the efficacy of the fermentation process using resting cells (washed).

A procedure similar to that described in Example 1 was employed, except that after an initial incubation period of 3 days, the cells from 100 mL of culture broth were harvested and washed (3 x 25 mL) with 0.3 x $10^{-4}$ M phosphate buffer (pH = 7.2) and separated by centrifugation. The washed cells were dispersed in the above mentioned buffer (100 mL) and incubated at 25 ± 1° C on a rotary shaker (rpm 200) for 7 days. Sclareol (0.5 g) dissolved in Tween-80 (5 g) was added incrementally to the suspension of cells during the first 4 days of incubation. At the end of the incubation period TLC monitoring indicated that all of the Sclareol (4) had been converted to the diol 3. Work-up,in the usual manner,provided diol 3 in 98% yield, and 99% GLC purity. The spectral data for this product was identical with that reported in Example 1.

EXAMPLE 4

This example demonstrates the fermentation process using each of Compounds 4 through 14 as

substrates, to produce decahydro-2-hydroxy-2,5,5,8a-tetramethylnaphthaleneethanol (3).

Eleven flasks, each containing an aqueous solution (100 mL) of medium described in Example 2 were sterilized at 120 °C for 20 minutes. A 50% aqueous solution of dextrose (4 mL), and Tween-80 (0.1 mL) containing 10 mg of corresponding substrate, was added to each flask. Each flask was then inoculated with 5% by volume of three days grown cells of CBS 214.83 (ATCC 20624) and the cultures were then incubated at 24 ± 1 °C on a rotary shaker (200 rpm) for 3 days. After the inital incubation period, a mixture of substrate dissolved in Tween-80 (ratio 1:7 w/w) was added to each corresponding flask, after which the incubation was continued for several days (see Table 4). The progress of the transformations was monitored by TLC. At the end of the incubation periods, the contents of each flask were extracted with ethyl acetate (3 x 75 mL), the extracts dried ($Na_2SO_4$), and the solvent evaporated. The residues were separately purified by column chromatography on silica gel using hexane/isopropane (95/5) as solvent, and the yields of diol 3 were determined. Data for the eleven experiments are summarized in Table 4.

Table 4

| Production of Compound 3 by CBS 214.83 (ATCC 20624) Using Different Substrates | | | |
|---|---|---|---|
| Substrate | (g/100 mL) | Total Incubation Time (days) | Yield of Diol 3 (%) |
| Compound 4 | 0.3 | 7 | 96 |
| Compound 5 | 0.3 | 11 | 89 |
| Compound 6 | 0.3 | 7 | 91 |
| Compound 7 | 0.2 | 10 | 51 |
| Compound 8 | 0.2 | 10 | 13 |
| Compound 9 | 0.2 | 10 | 7 |
| Compound 10 | 0.3 | 7 | 98 |
| Compound 11 | 0.3 | 6 | 100 |
| Compound 12 | 0.3 | 11 | 91 |
| Compound 13 | 0.24 | 8 | 98 |
| Compound 14 | 0.5 | 4 | 100 |

EXAMPLE 5

This example demonstrates a method for preparing Compounds 7, 8 and 9, which may be employed as substrates in the inventive process.

A solution of Sclareol (9.24 g, 0.03 mol) in methylene chloride (40 mL) was added in one portion to a mixture of pyridinium chlorochromate (12.93 g, 0.06 mol), sodium acetate (2.46 g, 0.03 mol), and methylene chloride (100 mL). The mixture was stirred for 4 h at 25 °C. Ether (200 mL) was added and the supernatant decanted from the gummy precipitate. The precipitate was washed with ether (3 x 50 mL). The ethereal solution was passed through silica gel 60 (40 g) and the solvents evaporated.

The residue was dissolved in ethanol (2 mL) and ether (4 mL), and stirred at 25 °C with a solution of sodium bisulfite (15 g) in water (60 mL) for 3 h. The mixture was extracted with ether (2 x 30 mL). The aqueous layer was made basic with 10% sodium hydroxide and extracted with ether (4 x 50 mL). The ether extracts were dried ($Na_2SO_4$) and the solvent evaporated to yield 2.72 g of residue. Chromatography on silica gel 60 (70 g; eluant, hexane: ethyl acetate; 4:1) gave 0.74 g of trans-aldehyde 7, 0.69 g of cis-aldehyde 8, and 0.64 g of cyclic aldehydes 9. [(E)-1R-(1α,2β,4aβ,-8aα)]-5-(decahydro-2-hydroxy-2,5,5,8a-tetramethyl-1-naphthalenyl)-3-methyl-2-pentenal (7) mp 83-85 °C, $[\alpha]_D$ + 14.2° (c,5.76, $CHCl_3$); $^1$H-NMR ($CDCl_3$) δ 0.78 (6H,s), 0.86 (3H, s), 1.17 (3H, s), 2.15 (3H, broad s), 0.8 - 2.5 (17H, m), 5.82 (1H, d, J = 8 Hz), 9.98 (1H, d, J = 8 Hz); IR ($CHCl_3$) $\nu_{max}$ 3570, 3440, 2940, 2850, 1670, 1630, 1460, 1440, 1390 $cm^{-1}$; MS, m/e 306, 291, 273, 109, 95, 84; UV $\lambda_{max}$ (95% ethanol) 241 nm (calcd 231 nm) (ε, 17,300). Anal. Calcd for $C_{20}H_{34}O_2$: C, 78.37; H, 11.18. Found: C, 77.92, H, 11.02. [(Z)-1R-(1α,2β,4aβ,8aα)]-5-(decahydro-2-hydroxy-2,5,5,8a-tetramethyl-1-naphthalenyl)-3-methyl-2-pentanal (8), mp 91-93.5 °C $[\alpha]_D$ + 8.9 (c, 3.13, $CHCl_3$); $^1$H-NMR ($CDCl_3$) δ 0.78 (6H, s), 0.87 (3H, s), 1.14 (3H, s), 1.98 (3H, broad s), 0.9 - 2.8 (17H, m), 5.75 (1H, d, J = 8 Hz), 10.0 (1H, d, J = 8 Hz); IR ($CHCl_3$) 3570, ˜450, 2940, 2840, 1670, 1630, 1460, 1440, 1390 $cm^{-1}$; MS m/e 306, 273, 109, 95, 84; UV $\lambda_{max}$ (95% ethanol) 242 nm (calcd 231 nm) (ε, 13,000). Anal. Calcd for $C_{20}H_{34}O_2$: C, 78.37; H, 11.18. Found : C, 78.34; H, 11.02. [4aR - (4aα,6aβ,10aα,10bβ)]-dodecahydro-3,4a,7,7,10a-pentamethyl-1H-naphtho[2,1-b]pyran-1-acetaldehyde (9), $^1$H-NMR ($CDCl_3$) δ 0.78

(6H, s), 0.85 (3H, s), 1.25 (3H, s), 1.27 (3H, s). 0.9 - 2.6 (18H, m), 9.8 - 10.0 (1H, m); IR (film) $\nu_{max}$ 2940, 2850, 1720, 1460, 1440, 1380, 1370 $cm^{-1}$; MS, m/e (two peaks of similar ms) 291, 273, 262, 245, 109, 43.

EXAMPLE 6

This example demonstrates a method for preparing Compound 10, which may be employed as a substrate in the invention process.

To a suspension of sodium hydride (0.72 g of 50% suspension, 0.015 mol; washed free of mineral oil with hexane) in dimethoxyethane (15 mL) was added a solution of ethyl diisopropylphosphononoacetate (3.78 g, 0.015 mol) in dimethoxyethane (30 mL) over a 10 min period. After hydrogen evolution has ceased [1R-(1α,2β,4aβ,8aα)]-4-(2-acetyloxy-decahydro-2,5,5,8a-tetramethyl-1-naphthalenyl)-2-butanone (3.22 g, 0.01 mol, which may be prepared as described by J.A. Barltrop et al., in J. Chem. Soc., 1960, 4613) was added all at once. The mixture was heated at reflux for 21 h, then cooled and poured onto ice water (100 mL). The mixture was acidified with 6N hydrochloric acid, and extracted with hexane/ethyl acetate (4:1, 4 x 10 mL). The organic extracts were washed with water (2 x 10 mL), saturated sodium bicarbonate solution (2 x 15 mL), and dried ($Na_2SO_4$). The solvents were evaporated and the residue chromatographed (silica gel 60; eluant, hexane : ethyl acetate, 9:1) to provide 3.02 g of ethyl [(E,Z)-1R-(1α,2β,4aβ,8aα)]-5-(2-acetyloxy-decahydro-2,5,5,8a-tetramethyl-1-naphthalenyl)-3-methyl-2-pentenoate as a colorless viscous oil.

A mixture of ethyl [(E,Z)-1R-(1α,2β,4aβ,8aα)]-5-(2-acetyloxy-decahydro-2,5,5,8a-tetramethyl-1-naphthalenyl)-3-methyl-2-pentenoate (2.19 g, 0.00557 mol), isopropanol (65 mL), water (10 mL) and potassium hydroxide (1.47 g, 0.0223 mol) was heated at reflux for 24 h. The mixture was concentrated to 20 mL, water (50 mL) was added, and the mixture was extracted with ether. The aqueous phase was acidified with 6N HCl and extracted with ether (5 x 20 mL). The ether extracts were washed with brine, dried ($Na_2SO_4$), and the solvents evaporated to yield 1.781 g of crude product. Chromatography on silica gel 60 (eluant, hexane : isoproanol, 9:1) gave 0.455 g of the cis-isomer, and 1.223 g of the trans-isomer 10. Recrystallization from hexane/ethyl acetate yielded analytical samples [(Z)-1R-[1α,2β,4aβ,8aα)]-5-(decahydro-2-hydroxy-2,5,5,8a-tetramethyl-1-naphthalenyl)-3-methyl-2-pentenoic acid, mp 147-149° C; $[\alpha]_D$ +62.96° (c, 4.66, $CHCl_3$); $^1$H-NMR ($CDCl_3$) δ 0.81 (6H, s), 0.88 (3H, s), 1.21 (3H, s), 1.92 (3H, broad s), 0.9 - 2.4 (17H, m), 5.72 (1H, broad s), 6.8 - 7.3 (1H, v. broad s); IR ($CHCl_3$) $\nu_{max}$ 3500, 3400, 2940, 2550, 1690, 1640, 1460, 1440 $cm^{-1}$; MS,m/e 322, 304, 289, 276, 109; UV $\lambda_{max}$ (95% EtOH) 228 nm (calcd 217 nm) (ε, 7300). Anal. Calcd for $C_{20}H_{34}O_3$: C, 74.49; H, 10.63. Found: C, 74.20; H, 10.40. [(E)-1R-(1α,2β,4aβ,8aα)]-5-(decahydro-2-hydroxy-2,5,5,8a-tetramethyl-1-naphthalenyl)-3-methyl-2-pentenoic acid, mp 151-153° C $[\alpha]_D$ 9.44° (c, 4.83, $CHCl_3$); $^1$H-NMR ($CDCl_3$) δ 0.79 (6H, s), 0.87 (3H, s), 1.15 (3H, s), 2.17 (3H, broad s) 0.9 - 2.4 (17H, m). 5.70 (1H, broad s), 5.8 - 6.1 (1H, broad s); IR ($CHCl_3$) $\nu_{max}$ 3560, 3400, 2940, 2550, 1690, 1640, 1460, 1440 $cm^{-1}$; MS, m/e 322, 304, 289, 276, 109; UV $\lambda_{max}$ (95% EtOH) 230 nm (calcd 217 nm) (ε, 5700). Anal. Calcd for $C_{20}H_{34}O_3$: C 74.49; H, 10.63. Found: C, 74.12; H, 10.52.

EXAMPLE 7

This example demonstrates a method for preparing Compound 11, which may be employed as a substrate in the inventive processs.

To a solution of diisopropylamine (8.484 g, 0.084 mol) in tetrahydrofuran (90 mL) at 0° C was added n-butyllithium (38.2 mL of a 2.2. M hexane solution, 0.084 mol) dropwise, over a 20 minute period. A solution of acetic acid (2.52 g, 0.042 mol) in tetrahydrofuran (20 mL) was added over a 15 minute period. The mixture was then heated at 50° C for 45 minutes. The mixture was cooled to 25° C and [1R-(1α,2β,4aβ,8aα)-4-(2-acetyloxydecahydro-2,5,5,8a-tetramethyl-1-naphthalenyl)-2-butanone (4.508 g, 0.014 mol, which may be prepared as described by J.A. Barltrop et al., in J. Chem. Soc., 1960, 4613) in tetrahydrofuran (35 mL) was added over a 10 minute period. The mixture was stirred at 25° C for 17 h and then heated at reflux for 30 minutes. The mixture was cooled to 25° C, afterwhich water (40 mL) and potassium hydroxide (5 g) were added. The mixture was heated at reflux for 4h, then cooled, added to water (100 mL), and extracted with hexane/ethyl acetate (4:1) (3 x 30 mL). The aqueous layer was cooled to 0° C, acidified with 6N HCl, and extracted with hexane/ethyl acetate (4:1, 4 x 50 mL). The combined extracts were washed with brine, dried ($Na_2SO_4$), and then the solvent evaporated to yield 2.071 g of crude product. Chromatograph on silica gel 60 (eluant, hexane : ethyl acetate: acetic acid, (10:10:0.1) gave 1.434 g of acids 11. Crystallization from hexane/ethyl acetate gave an analytical sample, mp 136-137.5° C, $^1$H-NMR ($CDCl_3$) δ 0.76 (6H, s), 0.85 (3H, s), 1.16 and 1.19 (3H, 2s), 1.28 (3H, s), 0.8 - 1.9 (16H, m), 2.4 - 2.8 (2H, m), 6.1 - 6.6 (2H, broad s); IR ($CHCl_3$) $\nu_{max}$ 3550, 2930, 2700, 1710, 1455, 1385 $cm^{-1}$; MS m/e 340, 304, 289, 109, 95, 43. Anal. Calcd for $C_{20}H_{36}O_4$:C, 70.54; H, 10.66. Found C, 70.99, H, 10.63.

EXAMPLE 8

This example demonstrates the conversion of α-ethenyldecahydro-2-hydroxy-α,2,5,5,8a-pentamethyl-1-napthalenepropanol (4) to dodecahydro-3a,6,6,9a-tetramethylnaphtho[2,1-b]furan (1) using a two-step process.

A procedure similar to that described in Example 2 was used, except that seven flasks were used, and that the amount of Sclareol (4) added to each flask and the incubation period was varied (see Table 5). When incubation was complete the flasks were worked-up separately and seven samples of crude diol 3 obtained. Each sample was separately reacted with toluene-p-sulfonylchloride, in pyridine, according to the method of Cambie, et al., (see Aust. J. Chem.,1971, 24, 591) and each reaction product Kugelrohr distilled to provide furan 1. $^1$H-NMR ($CDCl_3$) δ 0.83 (6H, 2s), 0.88 (3H, s), 0.9-1.8 (13H, m), 1.9-2.0 (1H, m), 3.77-3.92, (2H, m). IR (melt) $\nu_{max}$ 2940, 1460, 1385, 1365 $cm^{-1}$, MS m/e 236, 221, 204, 177, 137, 97. Data for the seven experiments are summarized in Table 5.

Table 5

| Flask | Wt. of Sclareol (4) mg | Total Incubation Time (days) | Product 1 *Yield (mg) | Product 1 GLC Purity (%) |
|---|---|---|---|---|
| 1 | 160 | 7 | 115 | 95 |
| 2 | 240 | 8 | 169 | 97 |
| 3 | 320 | 9 | 220 | 97 |
| 4 | 400 | 10 | 270 | 93 |
| 5 | 400 | 14 | 258 | 96 |
| 6 | 560 | 14 | 361 | 97 |
| 7 | 720 | 14 | 468 | 97 |

* Yields do not take into account samples removed during monitoring.

EXAMPLE 9

This example demonstrates the direct cyclization of the transformation product decahydro-2-hydroxy-2,5,5,8a-tetramethylnaphthaleneethanol (3), without separation from the aqueous nutrient medium, to dodecahydro-3a,6,6,9a-tetramethylnaphtho[2,1-b]furan (1).

A procedure similar to that described in Example 2 using Sclareol (2.0 g) as substrate in 100 mL of fermentation broth was employed. After 14 days total incubation time, the contents of the flask were transferred to a reaction vessel equipped for stirring and heating under reflux. Toluene-p-sulfonylchloride (2.48 g), sodium hydroxide pellets (25 g) and tetrahydrofuran (200 mL) were added and the mixture stirred at 20 °C. After 5 h,additional toluene-p-sulfonylchloride (1.50 g) was added and the reaction mixture was stirred overnight. Next day the mixture was heated at reflux for 30 min, cooled, and extracted with ethyl acetate (3 x 100 mL). The combined extracts were dried ($Na_2SO_4$). The solvents were evaporated, and the residue Kugelrohr distilled to give 1.38 g of a solid, which by instrumental analysis contained 85% of dodecahydro-3a,6,6,9a-tetramethylnaphtho[2,1-b]furan (1).

EXAMPLE 10

This example demonstrates an alternative procedure for the direct cyclization of the transformation product decahydro-2-hydroxy-2,5,5,8a-tetramethylnaphthaleneethanol (3), without separation from the aqueous nutrient medium, to dodecahydro-3a,6,6,9a-tetramethylnaphtho[2,1-b]furan (1).

A procedure similar to that described in Example 2 using Sclareol (2.0 g) as substrate in 100 mL of

fermentation broth was employed. After 14 days total incubation time, the contents of the flask were transferred to a reaction vessel equipped for stirring and heating under reflux. The fermentation broth was acidified with 6 N hydrochloric acid (to about pH 1), ethyl acetate (100 mL) added, and the stirred mixture heated at reflux for 6 h. After cooling, the ethyl acetate layer was separated, washed to neutrality, and dried. The solvent was evaporated and the residue Kugelrohr distilled to give 1.4 g of a solid, which by instrumental analysis contained 38% of dodecahydro-3a,6,6,9a-tetramethylnaphtho(2,1-b)furan (1).

EXAMPLE 11

This example illustrates an alternative procedure for the direct cyclization of the transformation product decahydro-2-hydroxy-2,5,5,8a-tetramethylnaphthaleneethanol (3), without separation from the aqueous nutrient medium, to dodecahydro-3a,6,6,9a-tetramethylnaphtho[2,1-b]furan (1).

A procedure similar to that described in Example 8 was employed except that the ion exchange resin Dowex 50X2 400 (10 g) was added to the fermentation broth instead of 6N hydrochloric acid. Work-up, and Kugelrohr distillation gave 1.32 g of a solid, which by instrumental analysis contained 37% of Compound 1.

The invention being thus described, it will be obvious that the same may be varied in many ways. Such variations are not to be regarded as a departure from the spirit and scope of the invention, and all such modifications are intended to be included within the scope of the following claims:

## Claims

1. A biologically pure culture of Hyphozyma roseoniger ATCC 20624.

2. A biologically pure culture of a Hyphozyma roseoniger microorganism or of a mutant thereof which has the identifying characteristic of Hyphozyma roseoniger ATCC 20624 of being capable of converting labdane compounds into recoverable quantities of a diol of the formula (3)

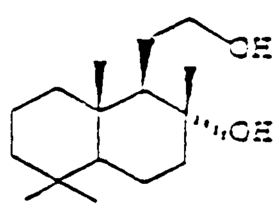

(3)

or into recoverable quantities of a furan of the formula (1)

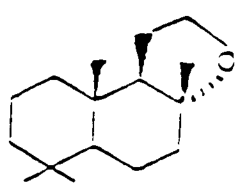

(1)

wherein the labdane compound is

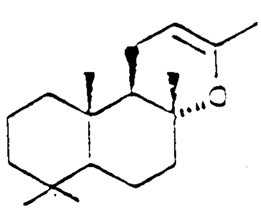

,

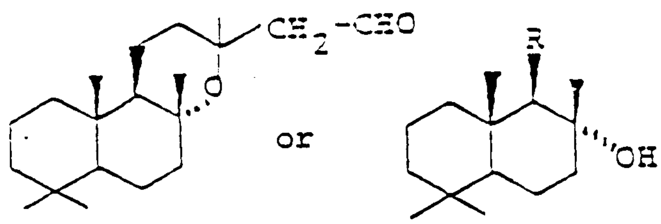

or

wherein R is

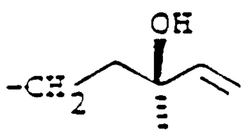
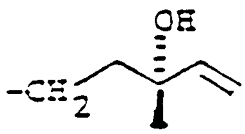
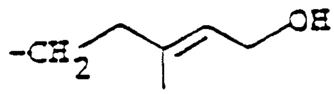

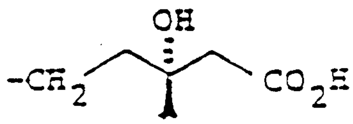
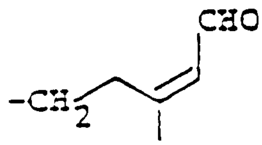
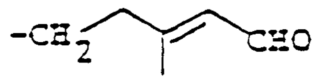

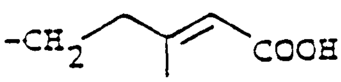
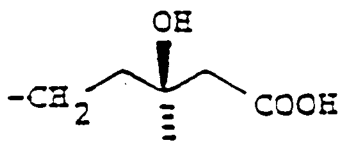

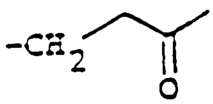
or
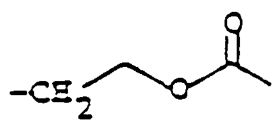
.

**3.** The biologically pure culture according to claim 1 or 2, characterized in that it is lyophylized.

**4.** A mixture obtained by cultivating a biologically pure culture according to any one of claims 1 to 3 under aerobic conditions in an aqueous medium.

**5.** A process for preparing a diol of the formula (3)

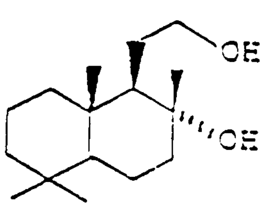
(3)

which comprises cultivating a biologically pure culture according to any one of claims 1 to 3 under aerobic conditions in an aqueous nutrient medium containing one or more of the compounds

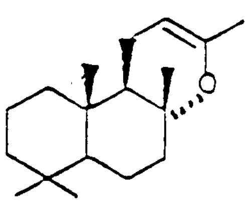
,
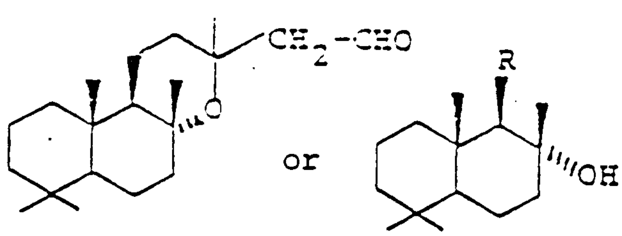
or

wherein R is

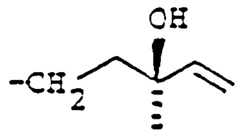 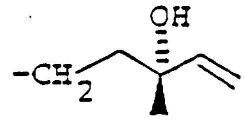 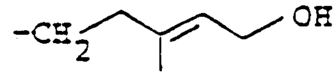

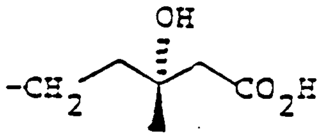 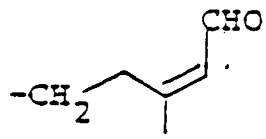 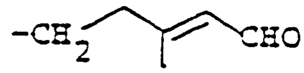

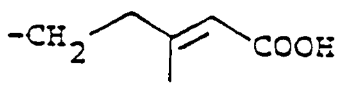 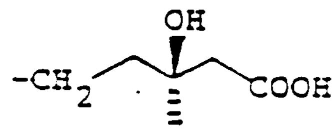

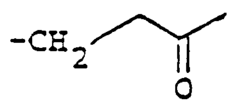 or 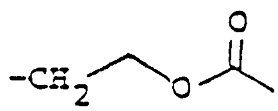 .

6. A process for preparing a furan compound of the formula (1)

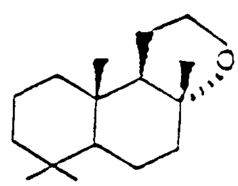 (1)

which comprises cultivating a biologically pure culture according to any one of claims 1 to 3 under aerobic conditions in an aqueous nutrient medium containing one or more of the compounds

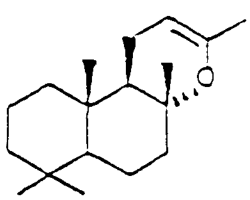 , 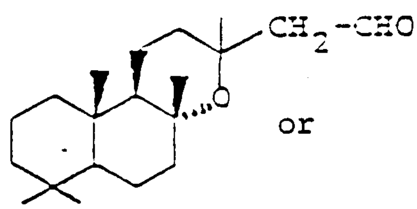 or 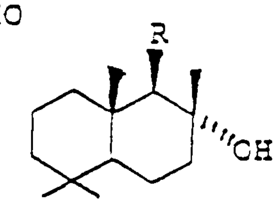

wherein R is

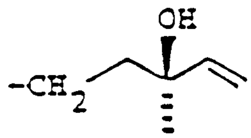

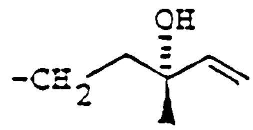

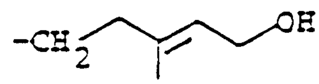

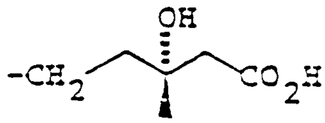

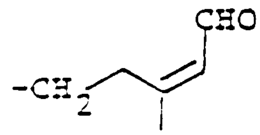

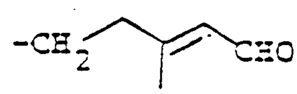

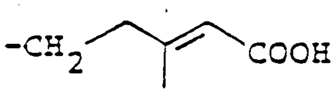

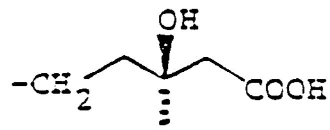

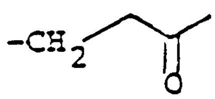

or

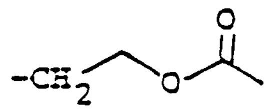

to form a diol of the formula (3)

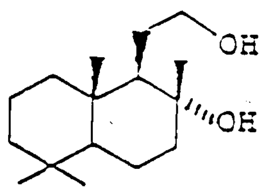

(3);

cyclizing the diol in the aqueous nutrient medium to form said furan compound; and recovering said furan compound from the nutrient medium.

7. A process for preparing a furan compound of the formula (1)

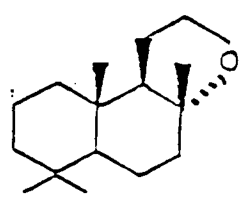

(1)

which comprises cultivating a biologically pure culture according to any one of claims 1 to 3 under aerobic conditions in an aqueous nutrient medium containing one or more of the compounds

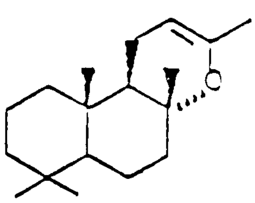

,

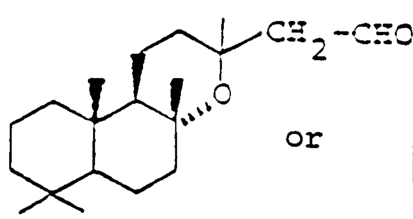

or

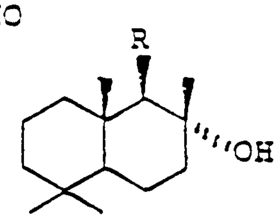

wherein R is

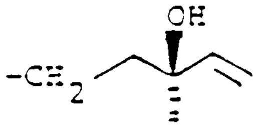

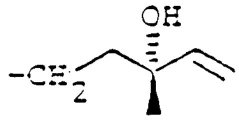

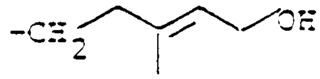

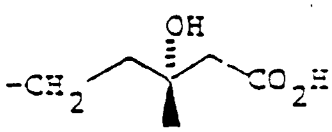

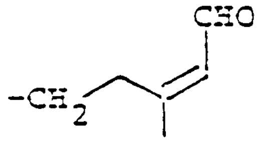

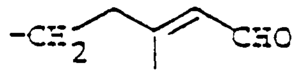

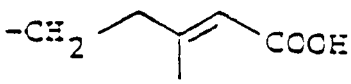

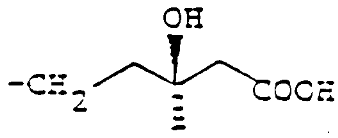

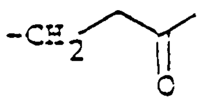

or

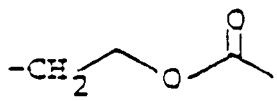

to form a diol having the formula (3)

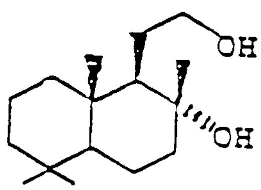

(3);

separating said diol; cyclizing said diol to form said furan compound; and recovering said furan compound.

## Revendications

1. Culture biologiquement pure de Hyphozyma roseoniger ATCC 20624.

2. Culture biologiquement pure d'un microorganisme appelé Hyphozyma roseoniger, ou un mutant de celui-ci, qui possède les caractéristiques d'identification de Hyphozyma roseoniger ATCC 20624 d'être capable de convertir des composés du type labdane en quantités récupérables d'un diol de la formule (3)

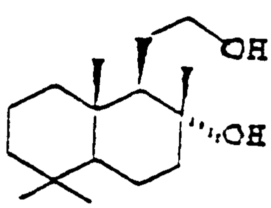

(3)

ou en quantités récupérables d'un furanne de la formule (1)

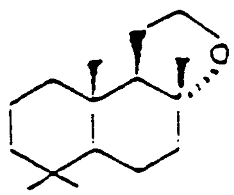

(1)

dans laquelle le composé du type labdane est le suivant

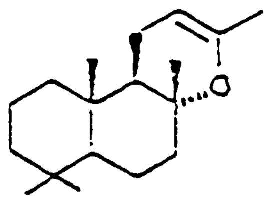

,

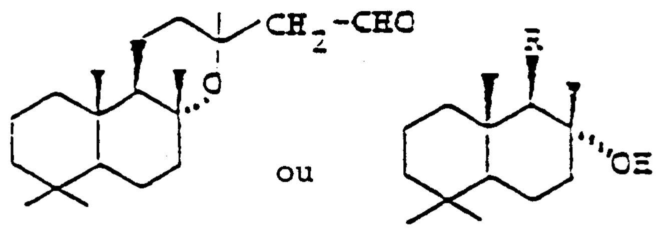

où R est

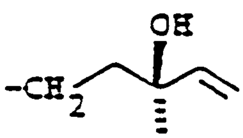

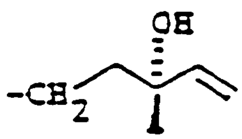

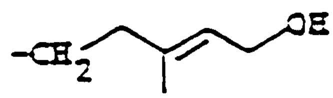

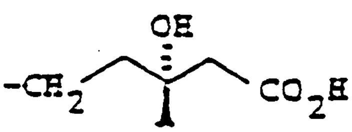

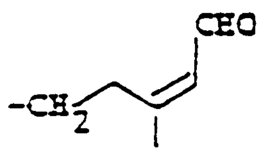

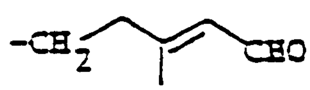

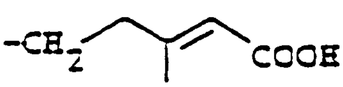

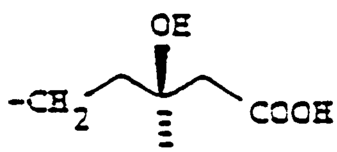

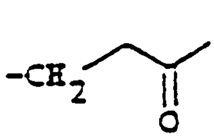

ou

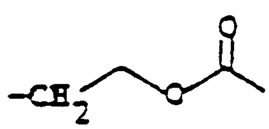

.

3. Culture biologiquement pure selon la revendication 1 ou 2, caractérisé en ce qu'elle est lyophilisée.

4. Mélange obtenu en cultivant une culture biologiquement pure suivant l'une quelconque des revendications 1 à 3 dans des conditions aérobies et dans un milieu aqueux.

5. Procédé de préparation d'un diol de la formule (3)

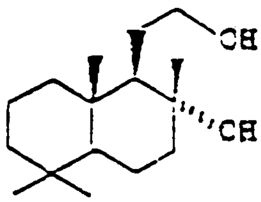

(3)

caractérisé en ce que l'on cultive une culture biologiquement pure selon l'une quelconque des revendications 1 à 3, dans des conditions aérobies et dans un milieu nutritif aqueux contenant un ou plusieurs des composés

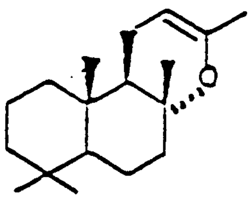

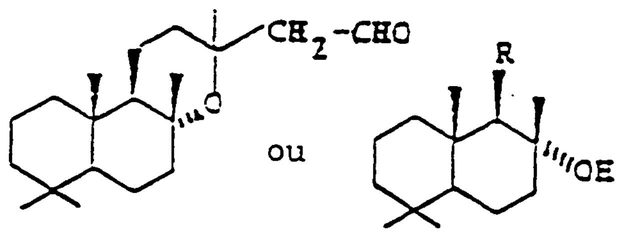

ou

où R est

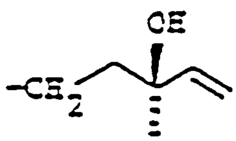

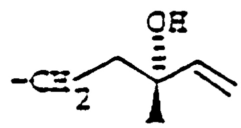

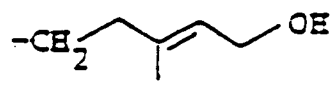

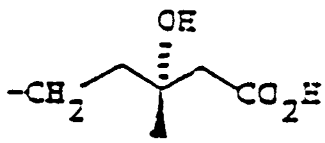

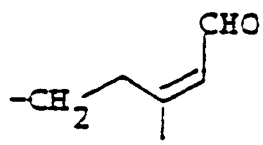

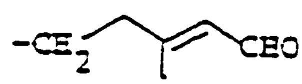

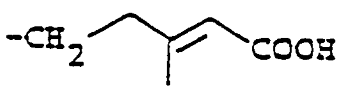

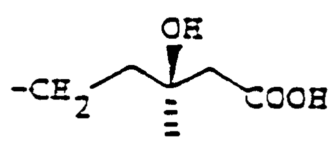

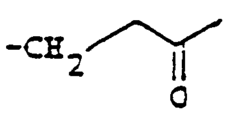

ou

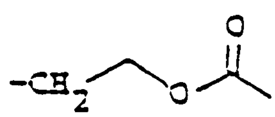

**6.** Procédé de préparation d'un composé de furanne de la formule (1)

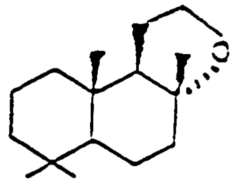

(1)

caractérisé en ce que l'on cultive une culture biologiquement pure selon l'une quelconque des revendications 1 à 3 dans des conditions aérobies et dans un milieu nutritif aqueux contenant un ou plusieurs des composes

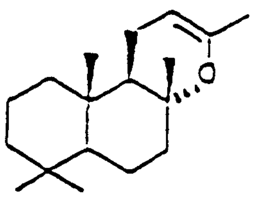

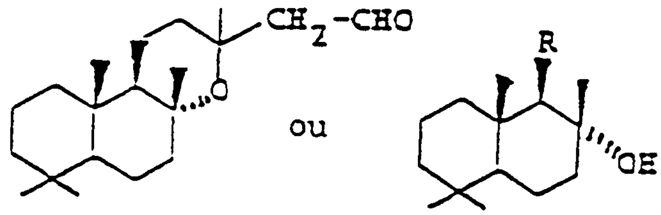

ou

où R est

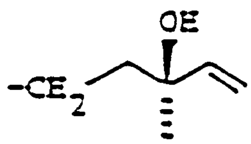 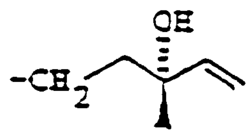 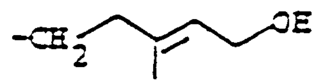

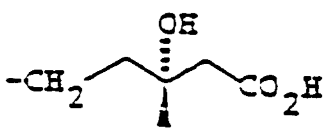 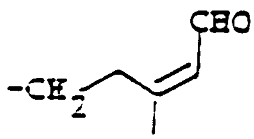 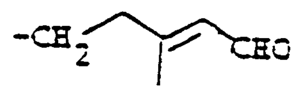

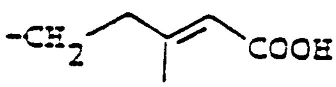 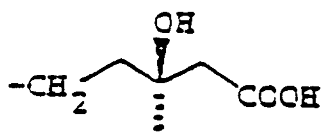

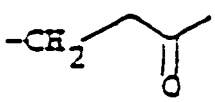 ou 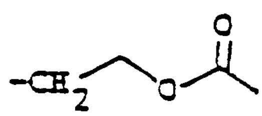

pour former un diol de la formule (3)

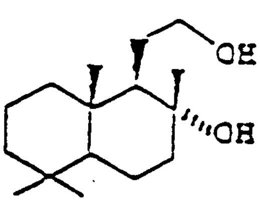

(3) ;

on cyclise le diol dans un milieu nutritif aqueux pour former le composé de furanne précité et on récupère ce composé de furanne du milieu nutritif.

**7.** Procédé de préparation d'un composé de furanne de la formule (1)

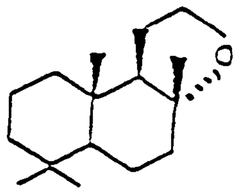

(1)

caractérisé en ce que l'on cultive une culture biologiquement pure selon l'une quelconque des revendications 1 à 3 dans des conditions aérobies et dans un milieu nutritif aqueux contenant un ou plusieurs des composés

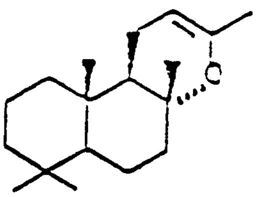

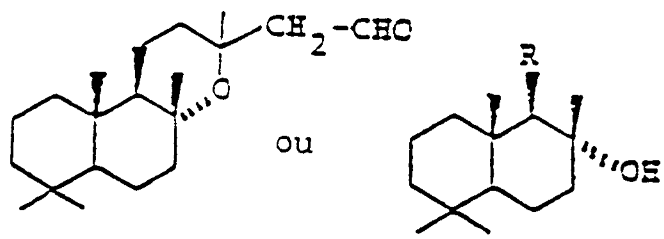

ou

où R est

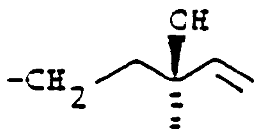

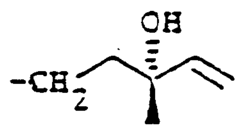

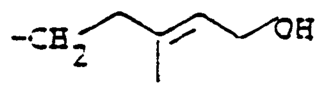

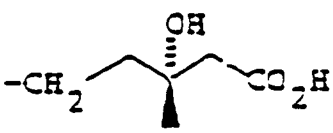

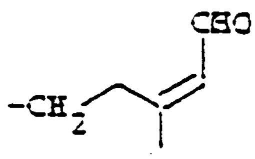

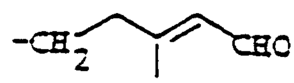

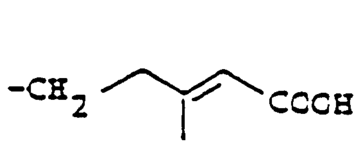

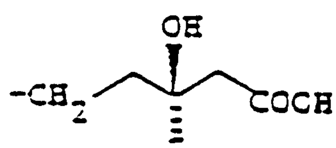

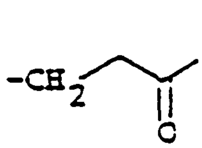

ou

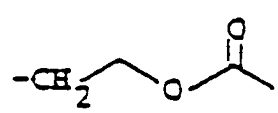

de manière à former un diol de la formule (3)

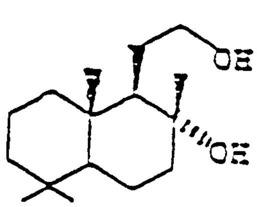

(3),

on sépare le diol précité, on cyclise le diol en question pour former le composé de furanne précité et on récupère ce composé de furanne.

## Patentansprüche

1. Biologische Reinkultur von Hyphozyma roseoniger ATCC 20624.

2. Biologische Reinkultur eines Mikroorganismus der Art Hyphozyma roseoniger oder einer Mutante davon, die als nachweisbares Merkmal von Hyphozyma roseoniger ATCC 20624 die Fähigkeit zur Umsetzung von Labdanverbindungen in gewinnbare Mengen eines Diols der Formel (3)

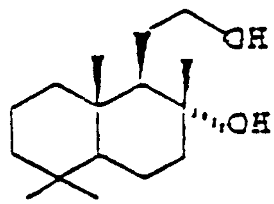

(3)

oder in gewinnbare Mengen eines Furans der Formel (1)

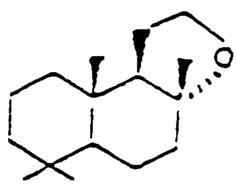

(1)

aufweist, wobei die Labdanverbindung

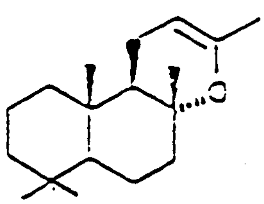

,

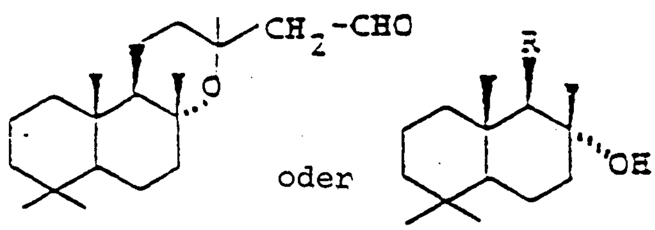

oder

ist, in der R

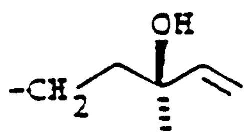
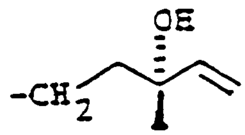
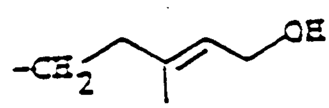

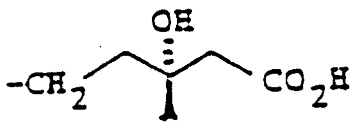
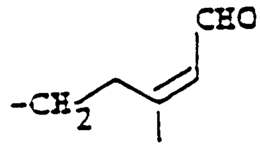
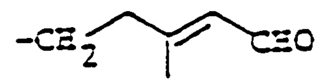

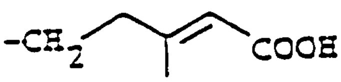
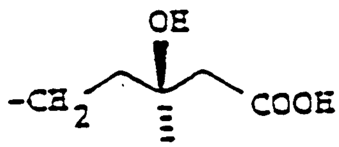

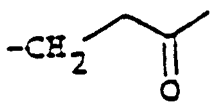
oder
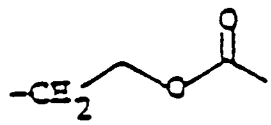

ist.

3. Biologische Reinkultur nach Anspruch 1 oder 2, dadurch gekennzeichnet, daß sie lyophylisiert ist.

4. Gemisch, erhalten durch Kultivierung einer biologischen Reinkultur nach einem der Ansprüche 1 bis 3 unter aeroben Bedingungen in einem wäßrigen Medium.

5. Verfahren zur Herstellung eines Diols der Formel (3)

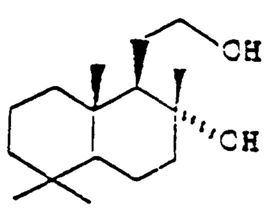
(3)

umfassend die Kultivierung einer biologischen Reinkultur nach einem der Ansprüche 1 bis 3 unter aeroben Bedingungen in einem wäßrigen Nährmedium, das eine oder mehrere der Verbindungen

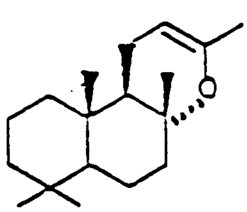
,
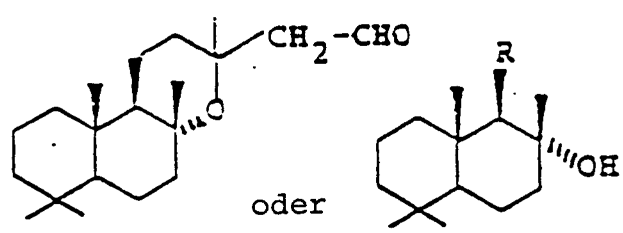
oder

enthält, in denen R

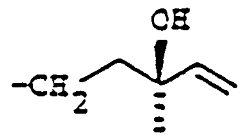 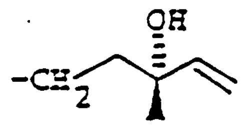 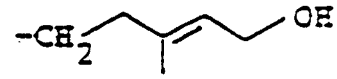

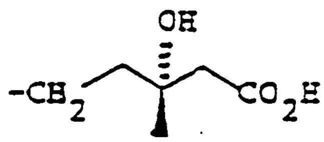 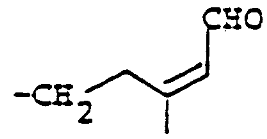 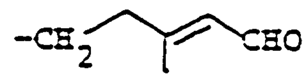

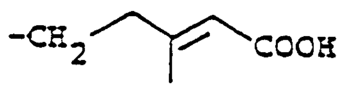 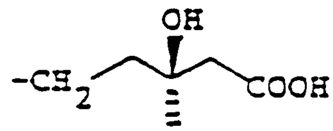

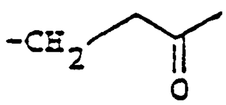 oder 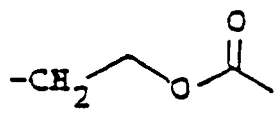

ist.

6. Verfahren zur Herstellung einer Furanverbindung der Formel (1)

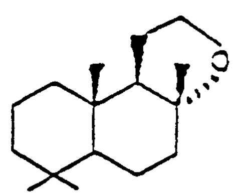 (1)

umfassend die Kultivierung einer biologischen Reinkultur nach einem der Ansprüche 1 bis 3 unter aeroben Bedingungen in einem wäßrigen Nährmedium, das eine oder mehrere der Verbindungen

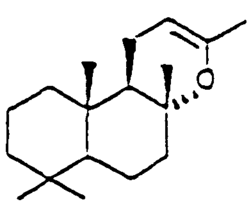 , 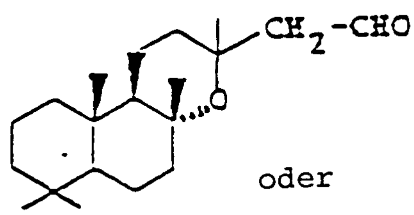 oder 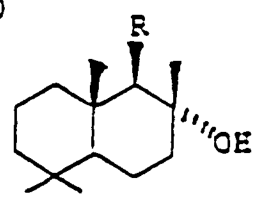

enthält, in denen R

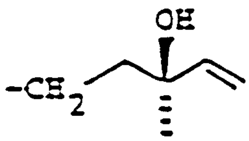 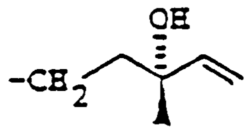 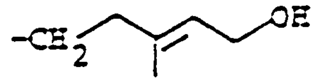

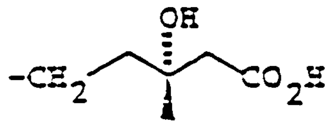 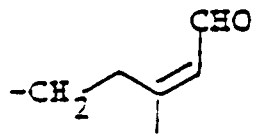 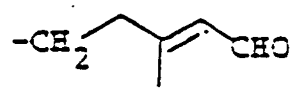

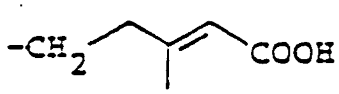 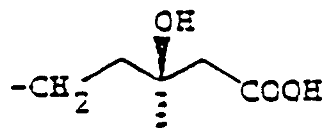

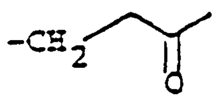 oder 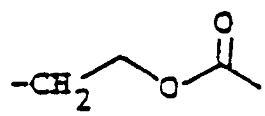

ist, zur Bildung eines Diols der Formel (3)

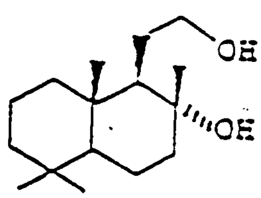 (3);

die Cyclisierung des Diols in dem wäßrigen Nährmedium zur Bildung der Furanverbindung, und die Gewinnung der Furanverbindung aus dem Nährmedium.

**7.** Verfahren zur Herstellung einer Furanverbindung der Formel (1)

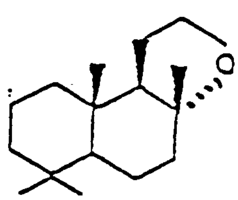 (1)

umfassend die Kultivierung einer biologischen Reinkultur nach einem der Ansprüche 1 bis 3 unter aeroben Bedingungen in einem wäßrigen Nährmedium, das eine oder mehrere der Verbindungen

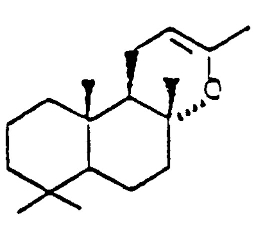 , 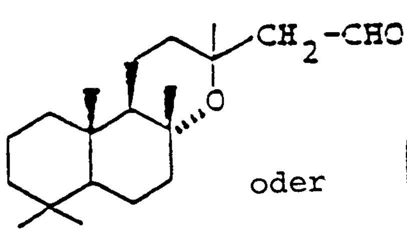 oder 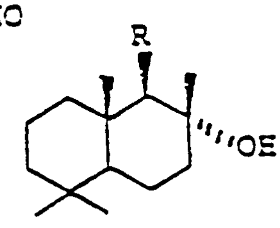

enthält, in denen R

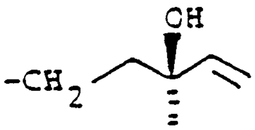
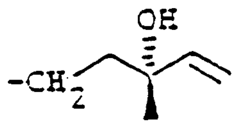
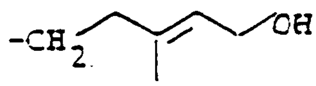
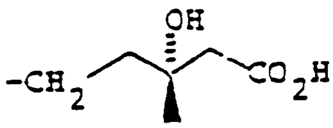
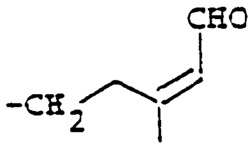
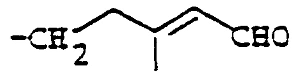
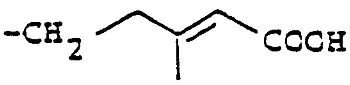
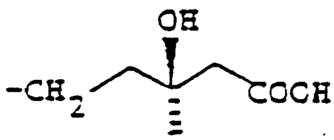
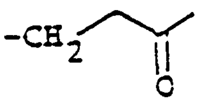
oder
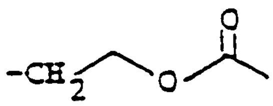

ist, zur Bildung eines Diols der Formel (3)

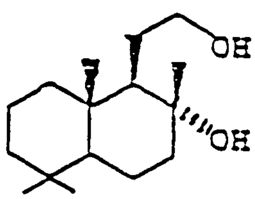
(3);

die Abtrennung des Diols, die Cyclisierung des Diols, zur Bildung der Furanverbindung, und die Gewinnung der Furanverbindung.